(19) Europäisches Patentamt
European Patent Office
Office européen des brevets

(11) EP 0 572 797 B1

(12) FASCICULE DE BREVET EUROPEEN

(45) Date de publication et mention de la délivrance du brevet: **08.01.1997 Bulletin 1997/02**

(21) Numéro de dépôt: **93106856.3**

(22) Date de dépôt: **28.04.1993**

(51) Int. Cl.$^6$: **C07C 33/14**, A61K 7/46, C07C 29/143, C07C 29/40, C07C 47/225, C07C 49/21

# (54) Alcools cycliques non-saturés leur utilisation à titre d'ingrédients parfumants et leurs intermédiaires.

Ungesättigte cyclische Alkohole, ihre Anwendung als Riechstoffbestandteile und deren Zwischenprodukte

Unsaturated cyclic alcohols, their use as perfume ingredients and their intermediates

(84) Etats contractants désignés:
**CH DE FR GB LI NL**

(30) Priorité: **02.06.1992 CH 1763/92**

(43) Date de publication de la demande:
**08.12.1993 Bulletin 1993/49**

(73) Titulaire: **FIRMENICH SA**
**CH-1211 Genève 8 (CH)**

(72) Inventeur: **Chapuis, Christian, Dr.**
**CH-1295 Mies (CH)**

(74) Mandataire: **Salvaterra-Garcia, Maria de Lurdes et al**
**Firmenich SA**
**Département des Brevets**
**Case Postale 239**
**1211 Genève 8 (CH)**

(56) Documents cités:
**EP-A- 0 155 591** **CH-A- 516 494**
**US-A- 4 278 098**

- **CHEMICAL ABSTRACTS CHEMICAL SUBSTANCE INDEX,12TH COLLECTIVE INDEX COLUMBUS OHIO; US page 28990CS**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 572 797 B1

Printed by Rank Xerox (UK) Business Services
2.13.11/3.4

## Description

La présente invention a trait au domaine de la parfumerie. Elle concerne, en particulier, des composés de formule

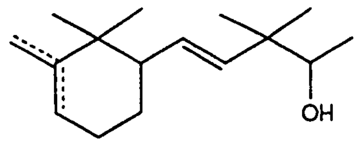

(I)

ayant une double liaison dans l'une des positions indiquées par les lignes pointillées.

Les composés de formule (I) sont des homologues structurels d'un alcool santalé très prisé en parfumerie, à savoir, le POLYSANTOL® [3,3-diméthyl-5-(2,2,3-triméthyl-3-cyclopentén-1-yl)-4-pentén-2-ol ; origine : Firmenich SA, Genève, Suisse]. Malgré l'abondance de composés parfumants de structure similaire à celle du POLYSANTOL®, c'est-à-dire possédant un cycle insaturé à 5 atomes de carbone, connus à cette date (voir, par exemple le brevet EP 155 591), nous n'avons trouvé dans la littérature aucune référence à des composés obéissant à une structure homologue, possédant un cycle insaturé à six atomes de carbone.

C'est donc de façon surprenante que nous avons maintenant découvert que les composés de formule (I) selon l'invention possédaient des propriétés odorantes utiles et, qui plus est, différaient de façon qualitative et quantitative de celles du POLYSANTOL® et autres composés analogues appréciés des parfumeurs.

Les composés de formule (I) possèdent en effet des propriétés odorantes qui se révèlent fort utiles, non seulement de par leur effet odorant de base de type santalé mais, et surtout, en raison du fait que leur note santalée se trouve associée à une note animale de type ambré, un caractère odorant qui est absent des notes odorantes des composés santalés homologues à cycle penténique, et possède l'avantage d'être à la fois santalée et cèdre-ambrée, contrairement à celle de ces derniers qui n'est que santalée.

Parmi les composés (I), c'est le composé possédant une double liaison endocyclique, ou 3,3-diméthyl-5-(2,2,3-triméthyl-3-cyclohexén-1-yl)-4-pentén-2-ol, qui développe au mieux les caractères odorants recherchés. Il possède une note santalée très puissante, très boisée, avec un côté santal naturel et une note boisée sèche, évoquant le bois de cèdre, accompagnée d'une sous-note ambrée. Ce composé a la préférence des parfumeurs dans les essais de comparaison avec des produits homologues commercialisés, essais qui sont décrits plus loin. Quant au composé (I) possédant une double liaison exocyclique, à savoir le 5-(2,2-diméthyl-3-méthylène-1-cyclohexyl)-3,3-diméthyl-4-pentén-2-ol, il possède une note boisée, santalée, douce, avec un aspect écorce et bois de santal. Quoique sa note soit moins puissante que celle du composé précédent et que celle du POLYSANTOL®, elle est plus douce et naturelle et ressemble plus à l'essence de santal naturelle de provenance Mysore, laquelle est remplacée avantageusement par les composés (I), comme il ressort des exemples présentés plus loin.

Les composés selon l'invention possèdent deux centres chiraux dans leur structure et peuvent ainsi assumer plusieurs formes isomériques optiquement actives. Bien que les mélanges racémiques ou optiquement actifs de deux ou plusieurs isomères soient des ingrédients parfumants avantageux, possédant les caractères odorants cités auparavant, les composés optiquement actifs de formule

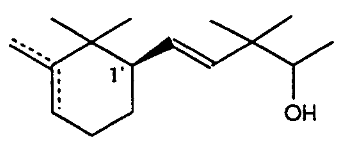

(Ia)

dans laquelle les lignes pointillées ont le sens indiqué à la formule (I), ainsi que leurs énantiomères correspondants, se sont aussi avérés être olfactivement intéressants et distincts entre eux.

Les composés selon la présente invention peuvent être utilisés aussi bien en parfumerie fine qu'en parfumerie fonctionnelle. Ils conviennent à la préparation de compositions parfumantes variées, de bases et concentrés parfumants, ainsi que parfums et eaux de toilette, auxquels ils impartissent des caractères santalés très naturels, ainsi que boisés-cèdre et ambrés. Leur application dans le parfumage d'articles divers tels les savons, gels de douche ou bain, shampoings, crèmes ou lotions après-shampoing, préparations cosmétiques ou désodorisants corporels ou d'air ambiant est aussi avantageuse.

Par ailleurs, ils conviennent également au parfumage de détergents ou adoucissants textiles, leurs notes odorantes étant d'une excellente tenacité, et de produits d'entretien.

Les proportions dans lesquelles les composés selon l'invention peuvent être incorporés dans les produits divers

susmentionnés varient dans une gamme de valeurs très étendue. Ces valeurs sont dépendantes de la nature du produit que l'on veut parfumer et de l'effet olfactif désiré, ainsi que de la nature des coingrédients dans une composition donnée, lorsque le composé (I) est utilisé en mélange avec des coingrédients parfumants, des solvants ou des adjuvants usuels dans l'art. Bien entendu, les composés selon l'invention peuvent également être ajoutés aux compositions et articles parfumés soit seuls, soit en solution dans des solvants d'usage courant.

A titre d'exemple on peut citer des concentrations de l'ordre de 1 à 5%, voire 10% ou plus en poids de composé selon l'invention, par rapport au poids de composition parfumante dans laquelle il est incorporé. Des concentrations bien inférieures à celles-ci peuvent être utilisées lorsque les composés de l'invention sont appliqués dans le parfumage des produits divers cités auparavant.

L'invention a également trait à un procédé pour la préparation d'un composé de formule (I) telle que définie auparavant, caractérisé en ce qu'on fait réagir un composé de formule

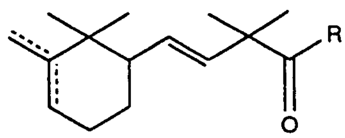

(II)

dans laquelle les lignes pointillées ont le sens indiqué à la formule (I) et R représente un atome d'hydrogène ou un radical méthyle, soit avec un agent réducteur, dans un solvant organique inerte, lorsque R est un radical méthyle, soit avec un halogéno-magnésien de méthyle lorsque R est un atome d'hydrogène.

Selon la première variante de ce procédé, la réaction s'effectue dans des conditions classiques de réduction d'une cétone en alcool et on peut utiliser en tant qu'agent réducteur n'importe quel réactif couramment employé dans ces conditions.

Selon l'autre variante du procédé de l'invention, on fait réagir un aldéhyde de formule (II) avec un méthyl-magnésien dans les conditions usuelles de la réaction de type Grignard.

Les produits de départ de formule (II) sont des composés nouveaux qui peuvent être préparés selon la méthode représentée schématiquement ci-après.

SCHEMA I

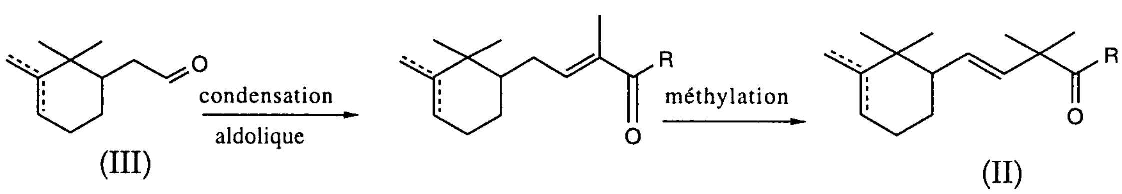


(III) (II)

- lignes pointillées représentent une double liaison dans l'une des positions indiquées
- R = H, $CH_3$

Les aldéhydes (III) utilisés comme produits de départ dans ce schéma sont des homologues de l'aldéhyde campholénique. Malgré le fait que des traces de l'un de ces composés, à savoir le 2,2,3-triméthyl-3-cyclohexène-1-acétaldéhyde, ont été détectées dans les huiles essentielles de plantes du genre *Sideritis* et *Eucaliptus* (voir, par exemple, V. Gergis et al., J. Sci. Food Agric. 1989, 47, 501), à notre connaissance, ce composé n'a jamais été préparé par voie de synthèse, ni caractérisé de façon complète. Ses isomères optiquement actifs cités ci-après sont d'ailleurs des composés de structure nouvelle. Les aldéhydes (III) sont préparés comme il est décrit dans les exemples présentés plus loin.

Les réactions de condensation aldolique, qui peuvent être des condensations de type aldéhyde-aldéhyde ou aldéhyde-cétone selon le composé de formule (II) que l'on désire obtenir, se déroulent dans les conditions courantes pour ce type de réaction, décrites en détail dans les exemples présentés ci-après. De même pour les réactions de méthylation subséquentes.

En raison du centre chiral présent dans la structure des aldéhydes (III), ces derniers peuvent assumer deux formes énantiomères qui mènent, suivant les réactions représentées au schéma I et le procédé de l'invention, à des composés

(II) sous des formes isomères optiquement actives de formule

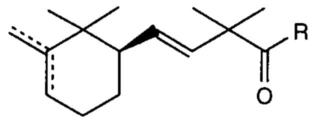

(IIa)

dans laquelle les lignes pointillées et le symbole R ont le sens indiqué à la formule (II), ou à leurs énantiomères correspondants.

Les composés (IIa), ou leurs énantiomères correspondants, permettent alors de préparer les composés de formule (Ia) mentionnés précédemment, ou respectivement leurs énantiomères correspondants, selon un mode d'exécution préférentiel du procédé de l'invention.

Cette dernière sera maintenant décrite plus en détail à l'aide des exemples de préparation suivants, dans lesquels les températures sont indiquées en degrés centigrades et les abréviations ont le sens usuel dans l'art.

L'invention sera également illustrée à l'aide des exemples d'application en parfumerie présentés plus loin.

Exemple 1

Préparation de (-)-(1'R,E)-3,3-diméthyl-5-(2',2',3'-triméthyl-3'-cyclohexén-1'-yl)-4-pentén-2-ol

a) (-)-(R)-2,2,3-triméthyl-3-cyclohexène-1-acétaldéhyde

Une solution de (-)-(1'R)-2-(2',2',3'-triméthyl-3'-cyclopentén-1'-yl)éthyl acétate [préparé comme il est décrit par K.-H. Schulte-Elte et al., Helv. Chim. Acta 1989, 72, 1158 ; $[\alpha]^{20}_D$ (pur) = - 1,53°, 395 g, 2,01 mole] dans le chlorure de méthylène (1000 ml) et méthanol (900 ml) a été refroidie à -40° et un courant d'ozone a été passé à travers la solution (18 g/h) jusqu'à disparition de tout le produit de départ. On a purgé avec $N_2$ et ajouté du diméthylsulfure (DMS, 400 ml) goutte à goutte, à -20°. Le mélange a été maintenu sous agitation pendant la nuit à 23° et ensuite concentré. L'huile brute a été diluée avec cyclohexane (500 ml) et on a ajouté de l'acide p-toluènesulfonique (10 g, 52,6 mmole). Le mélange a été porté à reflux pendant 4 h avec séparation continue d'eau. La solution refroidie a été lavée à l'eau, avec une solution aqueuse saturée de $Na_2CO_3$, eau et saumure, ensuite séchée sur $Na_2SO_4$ et évaporée. L'huile brute (402 g) a été distillée dans une colonne Vigreux de 12 cm pour fournir (+)-(1'R)-2-(6',6'-diméthyl-5'-oxo-3'-cyclohexén-1'-yl)éthyl acétate sous forme d'une huile incolore (pur à 99% ; rend. 65%).

$[\alpha]^{20}_D$ (pur) = + 56,2°
P. eb 85-89°/7,3 Pa
IR : 2950, 1720, 1660, 1460, 1420, 1380, 1360, 1230 $cm^{-1}$
RMN($^1$H, 360MHz) : 1,00(*s*, 3H) ; 1,18(*s*, 3H) ; 1,53(*m*, 1H) ; 1,93(*m*, 2H) ; 2,06(*s*, 3H) ; 2,17(*m*, 1H) ; 2,52(*dt*, $J_1$=7Hz, $J_2$=18Hz, 1H) ; 4,12(*m*, 2H) ; 5,97(*d*, *J*=9Hz, 1H) ; 6,84(m, 1H) δ ppm
RMN($^{13}$C) : 203,8(s) ; 170,8(s) ; 146,9(d) ; 128,2(d) ; 62,7(t) ; 45,1(s) ; 40,5(d) ; 28,7(t) ; 28,6(t) ; 22,3(q) ; 20,8(q) ; 18,9(q) δ ppm
SM : 210(1, $M^+$), 150(15), 135(9), 82(73), 68(100), 43(32).
Odeur : herbacée, légèrement foin, fleurie, légèrement boisée.

Une solution de cet acétate (33,6 g, 0,16 mole) dans l'éthanol (300 ml) a été hydrogénée à température ambiante et pression atmosphérique pendant 8 h (5 l $H_2$) sur du Ni Raney (1,4 g). Le mélange a été filtré, évaporé, séché sur $Na_2SO_4$ et distillé pour fournir 32,2 g (rend. 95%) de (+)-(1'R)-2-(2',2'-diméthyl-3'-oxo-1'-cyclohexyl)éthyl acétate (pur à 97%).

$[\alpha]^{20}_D$ (pur) = + 64,8°
P. eb 84°/6,7 Pa
IR : 2950, 1725, 1700, 1360, 1240 $cm^{-1}$
RMN($^1$H, 360MHz) : 1,04(*s*, 3H) ; 1,11(*s*, 3H) ; 1,45(*m*, 1H) ; 1,56(*m*, 2H) ; 1,62(*m*, 1H) ; 1,86(*m*, 2H) ; 2,00(*m*, 1H) ; 2,05(*s*, 3H) ; 2,31(*m*, 1H) ; 2,56(*m*, 1H) ; 4,04(*m*, 1H) ; 4,17(*m*, 1H) δ ppm
RMN($^{13}$C) : 215,3(s) ; 171,0(s) ; 63,2(t) ; 48,7(s) ; 44,5(d) ; 37,8(t) ; 29,1(t) ; 26,4(t) ; 25,0(t) ; 22,7(q) ; 20,9(q) ; 19,9(q) δ ppm
SM : 212(1, $M^+$), 152(13), 137(41), 124(45), 109(68), 96(42), 81(98), 67(49), 55(57), 43(100).

Une solution de ce composé (27 g, 0,127 mole) dans le toluène (500 ml) a été ajoutée goutte à goutte à une solution à reflux de KO(tert-butyl) (33,6 g, 0,3 mole) et iodure de méthyl triphénylphosphonium (121,2 g, 0,3 mole).

Après 3 h, le mélange refroidi a été versé sur glace et extrait à l'éther (4x100 ml), séché sur $Na_2SO_4$ et évaporé. L'huile brute (29,3 g) a été purifiée par chromatographie ($SiO_2$, 580 g, cyclohexane/acétate d'éthyle 8:2) pour fournir 19,1 g (rend. 72 %) de (+)-(1'R)-2-(2',2'-diméthyl-3'-méthylène-1'-cyclohexyl)éthyl acétate (pur à 98 %) sous forme d'une huile incolore.

$[\alpha]^{20}_D$ (pur) = + 58,8°
P. eb 115°/13,3 Pa
IR : 2900, 1705, 1600, 1410, 1330, 1200, 1000, 860 $cm^{-1}$
RMN($^1$H, 360MHz) : 0,95(*s*, 3H) ; 1,12(*s*, 3H) ; 1,33(*m*, 4H) ; 1,72(*m*, 2H) ; 1,86(*m*, 1H) ; 2,03(*s*, 3H) ; 2,20(*m*, 2H) ; 4,00(*m*, 1H) ; 4,10(*m*, 1H) ; 4,65(*s*, 2H) δ ppm
RMN($^{13}$C) : 171,1(s) ; 156,7(s) ; 105,9(t) ; 63,9(t) ; 43,9(d) ; 39,4(s) ; 33,1(t) ; 29,1(t) ; 27,5(t) ; 26,6(t) ; 26,2(q) ; 22,0(q) ; 21,0(q) δ ppm
SM : 210(0, $M^+$), 150(30), 135(70), 122(67), 107(100), 93(66), 79(86), 67(48), 55(35), 43(53).

Une solution de l'acétate obtenu en dernier lieu (7 g, 33,3 mmole) et d'acide p-toluènesulfonique (0,2 g, 1,16 mmole) dans le toluène (50 ml) a été portée à reflux pendant 2 h. La solution refroidie a été lavée successivement avec une solution aqueuse saturée de $NaHCO_3$ et de la saumure, et séchée sur $Na_2SO_4$. L'huile brute a été purifiée par chromatographie ($SiO_2$, 520 g, cyclohexane/acétate d'éthyle 9:1) pour fournir 4,84 g (rend. 70%) de (-)-(1'R)-2-(2',2',3'-triméthyl-3'-cyclohexén-1'-yl)éthyl acétate sous forme d'une huile incolore.

$[\alpha]^{20}_D$ (pur) = - 8,9°
P. eb 130°/13,3 Pa (temp. bain)
IR : 2900, 1700, 1410, 1325, 1200, 1000 $cm^{-1}$
RMN($^1$H, 360MHz) : 0,89(*s*, 3H) ; 1,02(*s*, 3H) ; 1,34(*m*, 3H) ; 1,64(*s*, 3H) ; 1,70(*m*, 1H) ; 1,88(*m*, 1H) ; 1,95(*m*, 2H) ; 2,05(*s*, 3H) ; 4,05(*m*, 1H) ; 4,19(*m*, 1H) ; 5,32(s large, 1H) δ ppm
RMN($^{13}$C) : 171,2(s) ; 140,9(s) ; 121,6(d) ; 64,0(t) ; 41,5(d) ; 37,1(s) ; 29,1(t) ; 26,0(q) ; 24,9(t) ; 23,7(t) ; 21,4(q) ; 21,0(q) ; 19,3(q) δ ppm
SM : 210(0, $M^+$), 150(23), 135(60), 121(19), 107(100), 96(32), 93(37), 81(53), 69(18), 55(14), 43(27).

A une suspension de $LiAlH_4$ (2,5 g, 0,066 mole) dans l'éther (400 ml) on a ajouté goutte à goutte à -10°, en 40 min., une solution de ce dernier acétate (20 g, 0,095 mole) dans l'éther (80 ml). Après 1 h à température ambiante, on a ajouté successivement, à 0°, de l'eau (3 ml), NaOH aqueux à 30% (3 ml) et eau (9 ml). Après 15 min, le mélange a été filtré sur Celite® et évaporé. On a distillé l'huile brute au four à boules (temp. bain : 100°/13,3 Pa) pour obtenir 15,2 g (rend. 95%) de (-)-(R)-2-(2',2',3'-triméthyl-3'-cyclohexén-1'-yl)-1-éthanol (pur à 99%).

$[\alpha]^{20}_D$ (pur) = - 12,8°
IR : 3300, 2950, 1440, 1360, 1050 $cm^{-1}$
RMN($^1$H, 360MHz) : 0,89(*s*, 3H) ; 1,03(*s*, 3H) ; 1,34(*m*, 4H) ; 1,50(*s*, 1H, +$D_2O$, OH) ; 1,65(*d*, *J*=2Hz, 3H) ; 1,81(*dt*, $J_1$=7Hz, $J_2$=9Hz, 1H) ; 1,95(*m*, 2H) ; 3,64(*m*, 1H) ; 1,75(*m*, 1H) ; 5,32(s large, 1H) δ ppm
RMN($^{13}$C) : 141,1(s) ; 121,6(d) ; 62,2(t) ; 41,2(d) ; 37,1(s) ; 33,3(t) ; 26,0(q) ; 25,1(t) ; 23,9(t) ; 21,4(q) ; 19,3(q) δ ppm
SM : 168(17, $M^+$), 150(9), 135(37), 123(62), 107(72), 96(53), 93(40), 81(100), 69(40), 55(29), 41(43).

A une suspension de chlorochromate de pyridinium (PCC, 35 g, 0,162 mole) et Celite® (50 g) dans du $CH_2Cl_2$ (450 ml) on a ajouté goutte à goutte une solution de l'alcool obtenu ci-dessus (16,6 g, 0,988 mole) dans 20 ml de $CH_2Cl_2$. Le mélange a été maintenu sous agitation pendant 1h à température ambiante, filtré sur Celite®, puis sur 100 g de $SiO_2$, avec éther, évaporé et distillé au four à boules (temp. bain : 100°/13,3 Pa) pour fournir 15,1 g (rend. 92%) du (-)-(R)-2,2,3-triméthyl-3-cyclohexène-1-acétaldéhyde (pur à 98%) désiré.

$[\alpha]^{20}_D$ (pur) = - 37,5°
IR : 2960, 2720, 1720, 1440, 1360 $cm^{-1}$
RMN($^1$H, 360MHz) : 0,90(*s*, 3H) ; 1,06(*s*, 3H) ; 1,43(*m*, 1H) ; 1,60(*m* 1H) ; 1,66(d, J=2Hz, 3H) ; 1,98(*m*, 2H) ; 2,03(*dt*, $J_1$=2Hz, $J_2$=8Hz, 1H) ; 2,21(*ddd*, $J_1$=2, $J_2$=8, $J_3$=15Hz, 1H) ; 2,58(*dd*, $J_1$=2, $J_2$=*15Hz, 1H*) ; 5,35(*s large*, 1H) ; 9,79(dd, $J_1$=1, $J_2$=3Hz, 1H) δ ppm
RMN($^{13}$C) : 203,2(d) ; 140,3(s) ; 121,7(d) ; 45,4(t) ; 39,2(d) ; 36,8(s) ; 26,4(q) ; 24,7(t) ; 24,4(t) ; 22,0(q) ; 19,3(q) δ ppm
SM : 166(11, $M^+$), 133(25), 121(39), 107(100), 96(26), 91(31), 81(62), 69(20), 55(18), 41(32).
Odeur : aldéhydée, verte, citron ; note de tête : grasse, camphre.

b) (-)-(1'R,E)-4-(2',2',3'-triméthyl-3'-cyclohexén-1'-yl)-2-méthyl-2-buténal

Dans un ballon, sous atmosphère de $N_2$, on a placé 0,470 ml de NaOH à 40% dans 15,66 ml de méthanol. On a chauffé à reflux, introduit en 1 h un mélange de 13 g (78,3 mmole) de l'aldéhyde obtenu selon a) et 156,6 mmole de propanal en maintenant le reflux jusqu'à la fin de la réaction. On a contrôlé par chromatographie en phase gazeuse la disparition de tout le produit de départ. On a laissé refroidir, ajouté 15,66 ml d'eau et glace. On a décanté la phase aqueuse, repris à l'éther de pétrole 30/50 et neutralisé à l'eau. On a séché, concentré, purifié par chromatographie sur $SiO_2$ avec cyclohexane/acétate d'éthyle 95:5 et distillé au four à boules (temp. bain : 120°/26,6 Pa) pour obtenir 7,7 g (rend. 47%) du buténal désiré (pur à 95%).

$[\alpha]^{20}_D$ (pur) = - 36,9°
IR : 2970, 1690, 1640, 1450, 1360, 1210, 1060, 800 $cm^{-1}$
RMN($^1$H, 360MHz) : 0,97(*s*, 3H) ; 1,1(*s*, 3H) ; 1,4(*m*, 1H) ; 1,53(*m*, 1H) ; 1,61(*m*, 1H) ; 1,66(d, J=2Hz, 3H) ; 1,77(*s*, 3H) ; 1,95(*m*, 2H) ; 2,18(*m*, 1H) ; 2,5(*m*, 1H) ; 5,35(s large, 1H) ; 6,55(t large, J=7Hz, 1H) ; 9,43(s, 1H) δ ppm
RMN($^{13}$C) : 9,36(q) ; 19,3(q) ; 21,7(q) ; 23,9(t) ; 24,8(t) ; 26,4(q) ; 29,9(t) ; 37,3(s) ; 45,0(d) ; 121,7(d) ; 140,1(s) ; 140,5(s) ; 155,2(d) ; 195,0(d) δ ppm
SM : 206(16, $M^+$), 173(15), 163(17), 136(50), 121(51), 107(62), 81(100), 41(41).
Odeur : grasse, aldéhydée.

c) (-)-(1'R,E)-3-méthyl-5-(2',2',3'-triméthyl-3'-cyclohexén-1'-yl)-3-pentén-2-one

Dans un ballon, sous atmosphère de $N_2$, on a dissous 17,8 mmole de NaOH dans 1,056 mole de méthanol. On a refroidi à -5° et ajouté 0,198 mole d'éthyl-méthyl-cétone. On a introduit 5,5 g (33 mmole) de l'aldéhyde obtenu selon a). Le mélange a été agité pendant 24 h à -5° et ensuite 24 h à température ambiante. On a ajouté 1,254 g de $H_2SO_4$ à 60%. Après avoir concentré le méthanol, on a repris à l'éther de pétrole 30/50 et neutralisé. On a séché, concentré et chromatographié sur colonne de $SiO_2$ avec cyclohexane/acétate d'éthyle 9:1 pour obtenir 6,5 g (rend. 89%) de la penténone désirée (pure à 99%).

$[\alpha]^{20}_D$ (pur) = - 33,12°
IR : 2970, 1690, 1590, 1440, 1360, 1280, 1080 $cm^{-1}$
RMN($^1$H, 360MHz) : 0,96(s) ; 1,10(s) ; 1,3-1,65(m, 4H) ; 1,67(*s*, 3H) ; 1,79(*s*, 3H) ; 1,95(m, 2H) ; 2,07(*m*, 1H) ; 2,33(*s*, 3H) ; 2,41(*m*, 1H) ; 5,35(*s* large, 1H) ; 6,68(t large, J=7Hz, 1H) δ ppm
RMN($^{13}$C) : 11,4(q) ; 19,3(q) ; 21,7(q) ; 23,9(t) ; 24,9(t) ; 25,5(q) ; 26,4(q) ; 30,0(t) ; 37,3(s) ; 45,1(d) ; 121,7(d) ; 138,3(s) ; 140,6(s) ; 144,2(d) ; 199,7(s) δ ppm
SM : 220(8, $M^+$), 205(5), 187(8), 177(15), 150(28), 135(23), 121(60), 107(58), 81(100), 43(85).
Odeur : légèrement santalée, légumineuse, choux.

d) (-)-(1'R)-3,3-diméthyl-5-(2',2',3'-triméthyl-3'-cyclohexén-1'-yl)-4-pentén-2- one

Dans un ballon séché, sous $N_2$, on a dissous 20,4 mmole de tert-butylate de potassium dans 11,9 ml de DMSO. On a amené la température à environ 20° et introduit 3,8 g (17 mmole) de la cétone préparé selon c). On a laissé réagir 30 min. On a refroidi à environ 5° et ajouté 20,4 mmole de $CH_3I$. On a laissé remonter la température, repris à l'éther de pétrole 30/50 et lavé à neutralité avec de l'eau. On a obtenu 2,8 g (rend. 70%) de la penténone désirée.

$[\alpha]^{20}_D$ (pur) = - 24,12°
IR : 2970, 1710, 1470, 1360, 1120, 980 $cm^{-1}$
RMN($^1$H, 360MHz) : 0,89(*s*, 3H) ; 0,99(*s*, 3H) ; 1,23(*s*, 6H) ; 1,58(*m*, 2H) ; 1,66(*d*, *J*=2Hz, 3H) ; 2,0(*m*, 3H) ; 2,12(*s*, 3H) ; 5,34(*s large*, 1H) ; 5,55(*m*, 2H) δ ppm
RMN($^{13}$C) : 19,2(q) ; 22,5(q) ; 24,2(q) ; 24,6(t) ; 25,4(t) ; 26,7(q) ; 37,1(s) ; 49,2(d) ; 50,3(s) ; 121,5(d) ; 132,5(d) ; 135,0(d) ; 140,7(s) ; 211,4(s)δ ppm
SM : 234(1, $M^+$), 191(22), 135(22), 121(65), 95(100), 81(78), 69(98), 55(29), 43(47).
Odeur : boisée, fruitée.

e) (-)-(1'R,E)-2,2-diméthyl-4-(2',2',3'-triméthyl-3'-cyclohexén-1'-yl)-3-buténal

On a procédé de façon analogue à celle décrite sous d) pour méthyler 2 g (9,7 mmole) de l'aldéhyde préparé selon b). Après chromatographie sur colonne de $SiO_2$, avec acétate d'éthyle/cyclohexane 3:97, on a obtenu 1,8 g (rend. 84%) du buténal désiré.

P. eb 120°/40 Pa (temp. bain)
$[\alpha]^{20}_D$ (pur) = - 22,15°

IR : 2970, 1730, 1465, 1360, 980 $cm^{-1}$

RMN($^1$H, 360MHz) : 9,35(*s*, 1H) ; 5,55(*dd*, $J_1$=8, $J_2$=13Hz, 1H) ; 5,38(*d*, *J*=13Hz, 1H) ; 5,32(*s large*, 1H) ; 2,0(*m*, 3H) ; 1,67(*s*, 3H) ; 1,58(*m*, 2H) ; 1,19(*s*, 6H) ; 0,98(*s*, 3H) ; 0,85(*s*, 3H) δ ppm

SM : 220(1, $M^+$), 121(11), 96(100), 81(50), 69(17), 55(12), 41(23).

f) Dans un ballon, sous $N_2$, on a dissous 8 mmole de $LiAlH_4$ dans 12 ml de tétrahydrofuranne (THF). On a introduit goutte à goutte 1,87 g (8 mmole) de la penténone préparée selon d) dilués dans 50 ml de THF, en maintenant la température entre 20° et 30°. On a laissé réagir 1 h. Après avoir concentré le THF, on a repris à l'éther de pétrole 30/50, lavé à neutralité avec HCl à 15%, séché, concentré et distillé pour obtenir 1,8 g (rend. 95%) de (-)-(1'R,E)-3,3-diméthyl-5-(2',2',3'-triméthyl-3'-cyclohexén-1'-yl)-4-pentén-2-ol (pur à 97%).

Le même composé a été obtenu en faisant réagir l'aldéhyde préparé selon e) avec un méthyl-magnésien, par exemple, $CH_3MgI$.

$[\alpha]^{20}_D$ (pur) = - 20,08°

IR : 3400, 2970, 1465, 1360, 1095, 980, 910 $cm^{-1}$

RMN($^1$H, 360MHz) : 0,89(*s*, 3H) ; 1,0(*s*, 9H) ; 1,11(*d*,*J*=7Hz, 3H) ; 1,58(*m*, 2H) ; 1,66(*s large*, 3H) ; 2,0(*m*, 3H) ; 3,5(*m*, 1H) ; 5,33(*s large*, 1H) ; 5,46(*m*, 2H) δ ppm

RMN($^{13}$C) : 17,5(q) ; 19,3(q) ; 22,3(q) ; 22,5(q) ; 23,9(q) ; 24,7(t) ; 25,7(t) ; 26,7(q) ; 36,9(s) ; 40,9(s) ; 49,3(d) ; 74,4(d) ; 121,5(d) ; 131,5(d) ; 137,4(d) ; 140,9(s) δ ppm

SM : 236(0, $M^+$), 192(11), 135(10), 121(45), 96(100), 81(78), 69(44), 41(28).

Odeur : décrite plus haut.

## Exemple 2

### Préparation de (+)-(1'R,E)-5-(2',2'-diméthyl-3'-méthylène-1'-cyclohexyl)-3,3-diméthyl-4-pentén-2-ol

a) (+)-(R)-2,2-diméthyl-3-méthylène-1-cyclohexaneacetaldéhyde

A une suspension de $LiAlH_4$ (0,4 g, 10,5 mmole) dans l'éther (50 ml) on a ajouté goutte à goutte à -10° une solution de (+)-(1'R)-2-(2',2'-diméthyl-3'-methylène-1'-cyclohexyl)éthyl acétate (voir Exemple 1a) ; 3,6 g, 17,1 mmole) dans l'éther (20 ml). Après 1 h à température ambiante, on a ajouté successivement, à 0°, de l'eau (0,4 ml), NaOH aqueux à 15% (0,4 ml) et eau (1,2 ml). Le mélange a été filtré sur Celite® et évaporé. On a distillé l'huile brute (2,9 g) dans un four à boules (temp. bain : 100°/13,3 Pa) pour obtenir 2,77 g (rend. 97%) de (+)-(1'R)-2-(2',2'-diméthyl-3'-méthylène-1'-cyclohexyl)-1-éthanol sous forme d'une huile incolore.

$[\alpha]^{20}_D$ (pur) = + 69°

IR : 3300, 2920, 1630, 1440, 1380, 1160, 1050, 890 $cm^{-1}$

RMN($^1$H, 360MHz) : 0,96(*s*, 3H) ; 1,13(*s*, 3H) ; 1,3(*m*, 4H) ; 1,45(*s large*, 1H,+$D_2$O-OH) ; 1,74(*m*, 2H) ; 1,79(*m*, 1H) ; 2,2(*m*, 2H) ; 3,59(*m*, 1H) ; 3,69(*m*, 1H) ; 4,65(*s*, 2H) δ ppm

RMN($^{13}$C) : 157,0(s) ; 105,7(t) ; 62,2(t) ; 43,7(d) ; 39,4(s) ; 33,4(t) ; 33,2(t) ; 27,7(t) ; 26,8(t) ; 26,2(q) ; 22,0(q) δ ppm

SM : 168(8, $M^+$), 153(11), 135(41), 123(61), 107(100), 93(45), 79(99), 67(94), 55(70), 41(68).

Odeur : vaguement camphrée, verte, chimique.

A une suspension de chlorochromate de pyridinium (PCC, 30 g, 0,139 mole) et 45 g de Celite® dans $CH_2Cl_2$ (300 ml) on a ajouté goutte à goutte, sur 1 h, une solution de l'alcool obtenu ci-dessus (15,5 g, 0,092 mole) dans $CH_2Cl_2$ (100 ml). Le mélange a été maintenu sous agitation pendant 1h à température ambiante. On a filtré sur $SiO_2$ (150 g), lavé à l'éther et évaporé pour obtenir 16,3 g d'huile brute. Celle-ci a été distillée au four à boules (temp. bain : 100°/13,3 Pa) pour fournir 15,2 g (rend. 99%) de l'acétaldéhyde désiré, pur à 97%.

$[\alpha]^{20}_D$ (pur) = + 20,5°

IR : 2940, 1720, 1630, 890 $cm^{-1}$

RMN($^1$H, 360MHz) : 0,96(*s*, 3H) ; 1,14(*s*, 3H) ; 1,40(*m*, 2H) ; 1,70(*m*, 2H) ; 1,94(*m*, 1H) ; 2,15(*m*, 1H) ; 2,22(*m*, 2H) ; 2,57(*m*, 1H) ; 4,70(*d*, *J=7Hz*, 2H) ; 9,74(*t*, J= 2Hz, 1H) δ ppm

RMN($^{13}$C) : 202,7(d) ; 155,6(s) ; 106,6(t) ; 45,6(t) ; 41,3(d) ; 39,1(s) ; 32,9(t) ; 28,6(t) ; 26,4(q) ; 26,2(t) ; 22,5(q) δ ppm

SM : 166(4, $M^+$), 151(10), 133(45), 122(61), 107(100), 91(35), 79(58), 67(50), 55(40), 41(49).

Odeur : verte, aldéhydée, menthée.

b) (+)-(1'R,E)-5-(2',2'-diméthyl-3'-méthylène-1'-cyclohexyl)-3-méthyl-3-pentén-2-one

On a procédé de façon analogue à celle décrite dans l'Exemple 1c) en utilisant 22,68 mmole de NaOH dans 1,344 mole de méthanol, 0,252 mole d'éthyl-méthyl-cétone, 7 g (42 mmole) de l'aldéhyde préparé selon a) ci-dessus et 1,596 g de $H_2SO_4$. On a obtenu 3,99 g (rend. 43%) de la penténone désirée (pure à 97%), après chromatographie sur $SiO_2$ avec toluène/acétate d'éthyle 97:3.

$[\alpha]^{20}_D$ (pur) = + 32,35°
IR : 2930, 1670, 1640, 1440, 1360, 1280, 1160, 1080, 890cm$^{-1}$
RMN($^1$H, 360MHz) : 1,03(*s*, 3H) ; 1,99(*s*, 3H) ; 1,35(*m*, 3H) ; 1,75(*m*, 2H) ; 1,78(*s*, 3H) ; 2,05(*dt*, $J_1$=9, $J_2$=15Hz, 1H) ; 2,21(*m*, 2H) ; 2,3(*s*, 3H) ; 2,4(*m*, 1H) ; 4,68(*s*, 2H) ; 6,63(*t*, J= 7Hz, 1H) δ ppm
RMN($^{13}$C) : 11,3(q) ; 22,2(q) ; 25,4(q) ; 26,5(q) ; 26,6(t) ; 27,7(t) ; 30,0(t) ; 33,0(t) ; 39,6(s) ; 47,4(d) ; 106,1(t) ; 138,0(s) ; 144,0(d) ; 156,3(s) ; 199,6(s) δ ppm
SM : 220(4, $M^+$), 205(5), 148(29), 133(31), 123(50), 107(26), 98(36), 93(30), 81(100), 67(50).
Odeur : santalée, laiteuse.

c) <u>(+)-(1'R,E)-5-(2',2'-diméthyl-3'-méthylène-1'-cyclohexyl)-3,3-diméthyl-4 pentén-2-one</u>

Suivant une méthode analogue à celle décrite dans l'Exemple 1d) on a méthylé 3,9 g (17,7 mmole) de la cétone préparée selon b) ci-dessus, à l'aide de 21,24 mmole de KO(tert-butyl) dans 12,4 ml de DMSO, et 21,24 mmole de $CH_3I$. Après chromatographie sur $SiO_2$ avec cyclohexane/acétate d'éthyle 95:5, on a obtenu 3,42 g (rend. 83%) de la penténone désirée (pure à 98,7%).

P. eb 100°/13,3 Pa (temp. bain)
$[\alpha]^{20}_D$ (pur) = + 55,32°
IR : 2960, 2930, 1710, 1640, 1470, 1360, 1120, 980, 890 cm$^{-1}$
RMN($^1$H, 360MHz) : 0,95(*s*, 3H) ; 1,06(*s*, 3H) ; 1,21(*s*, 3H) ; 1,22(*s*, 3H) ; 1,30-1,95(*m*, 5H) ; 2,1(*s*, 3H) ; 2,2(*m*, 2H) ; 4,64(*s*, 1H) ; 4,65(*s*, 1H) ; 5,5(*m*, 2H) δ ppm
RMN($^{13}$C) : 22,5(q) ; 24,2(q) ; 25,4(q) ; 26,7(t) ; 26,8(q) ; 29,1(t) ; 33,0(t) ; 39,3(s) ; 50,3(s) ; 51,7(d) ; 105,9(t) ; 132,1(d) ; 135,2(d) ; 156,3(s) ; 211,3(s) δ ppm
SM : 234(1, $M^+$), 191(14), 148(9), 135(19), 121(21), 109(23), 95(27), 69(100), 43(23).

d) On a procédé de façon analogue à celle décrite dans l'Exemple 1f), en utilisant 11 mmole de $LiAlH_4$ dans 16,5 ml de THF et 2,6 g (11 mmole) de la cétone préparée selon c) ci-dessus. Après chromatographie sur $SiO_2$ avec toluène/acétate d'éthyle 9:1, on a obtenu 1,83 g (rend. 70%) de (+)-(1'R,E)-5-(2',2'-diméthyl-3'-méthylène-1-cyclohexyl)-3,3-diméthyl-4-pentén-2-ol (pur à 97%).

P. eb 80°/6,7 Pa (temp. bain)
$[\alpha]^{20}_D$ (pur) = + 55,74°
IR : 3400, 2970, 2950, 1640, 1450, 1380, 1070 cm$^{-1}$
RMN($^1$H, 360MHz) : 0,96(*s*, 3H) ; 0,99(*s*, 6H) ; 1,07(*s*, 3H) ; 1,11(d, J=7Hz, 3H) ; 1,3-1,9(*m*, 5H) ; 2,2(*m*, 2H) ; 3,48(*m*, 1H) ; 4,65(d, J=3Hz, 2H) ; 5,4(*m*, 2H) δ ppm
RMN($^{13}$C) : 17,6(q) ; 22,5(2x q) ; 23,8(q) ; 26,8(t) ; 26,9(q) ; 29,5(t) ; 33,0(t) ; 39,1(s) ; 40,9(s) ; 51,8(d) ; 74,4(d) ; 105,7(t) ; 130,9(d) ; 137,6(d) ; 156,6(s) δ ppm
SM : 236(0, $M^+$), 218(1), 192(18), 149(19), 135(21), 121(25), 109(28), 93(46), 69(100), 55(27).
Odeur : décrite plus haut.

<u>Exemple 3</u>

<u>Préparation de (+)-(1'S, E)-3,3-diméthyl-5-(2',2',3'-triméthyl-3'-cyclohexén-1'-yl)-4-pentén-2-ol</u>

Ce composé a été préparé de façon analogue à celle décrite dans l'Exemple 1, mais en partant de l'énantiomère de l'acétate utilisé comme produit de départ dans l'Exemple 1 a). Cet acétate a été préparé de façon analogue à celle décrite par K.-H. Schulte-Elte et al., Helv. Chim. Acta <u>1989</u>, 1158, en partant de l'isomère approprié de l'aldéhyde campholénique ayant $[\alpha]^{20}_D$ (pur) = -9,6°.
Les données analytiques des composés obtenus étaient identiques à celles de leurs énantiomères correspondants décrits dans l'Exemple 1, à l'exception des angles de rotation optique, présentés ci-après.

<u>(+)-(1'S)-2-(2',2',3'-triméthyl-3'-cyclopentén-1'-yl)éthyl acétate</u>

$[\alpha]^{20}_D$ (pur) = + 1,2°

(-)-(1'S)-2-(6,'6'-diméthyl-5'-oxo-3'-cyclohexén-1'-yl)éthyl acétate

$[\alpha]^{20}_{D}$ (pur) = - 62,5°

(-)-(1'S)-2-(2',2'-diméthyl-3'-oxo-1'-cyclohexyl)éthyl acétate

$[\alpha]^{20}_{D}$ (pur) = - 71,2°

Cet acétate a été transformé en l'isomère correspondant du 2-(2',2'-diméthyl-3'-méthylène-1'-cyclohexyl)éthyl acétate selon le procédé décrit à l'Exemple 1a), lequel isomère, sans avoir été isolé, fut transformé selon l'Exemple 2a), 1er paragraphe, en l'alcool correspondant presenté ci-après :

(-)-(S)-2-(2',2'-diméthyl-3'-méthylène-1'-cyclohexyl)-1-éthanol

$[\alpha]^{20}_{D}$ (pur) = - 74,4°

Cet alcool a ensuite été transformé en son isomère de liaison de façon analogue à celle décrite dans l'Exemple 1a) pour les acétates correspondants.

(+)-(S)-2-(2',2',3'-triméthyl-3'-cyclohexén-1'-yl)-1-éthanol

$[\alpha]^{20}_{D}$ (pur) = + 12,7°

(+)-(S)-2,2,3-triméthyl-3-cyclohexène-1-acétaldéhyde

$[\alpha]^{20}_{D}$ (pur) = + 38,3°

(+)-(1'S,E)-3-méthyl-5-(2',2',3'-triméthyl-3'-cyclohexén-1'-yl)-3-pentén-2-one

$[\alpha]^{20}_{D}$ (pur) = + 29,6°

(+)-(1'S,E)-3,3-diméthyl-5-(2',2',3'-triméthyl-3'-cyclohexén-1'-yl)-4-pentén-2-one

$[\alpha]^{20}_{D}$ (pur) = + 24,9°

(+)-(1'S,E)-3,3-diméthyl-5-(2',2',3'-triméthyl-3'-cyclohexén-1'-yl)-4-pentén-2-ol

$[\alpha]^{20}_{D}$ (pur) = + 20,7°

Exemple 4

Préparation de (-)-(1'S,E)-5-(2',2'-diméthyl-3'-méthylène-1'-cyclohexyl)-3,3-diméthyl-4-pentén-2-ol

Ce composé a été préparé de façon analogue à celle décrite dans l'Exemple 2, à partir de (-)-(S)-2-(2',2'-diméthyl-3'-méthylène-1-'cyclohexyl)-1-éthanol ($[\alpha]^{20}_{D}$ (pur) = - 74,4° ; décrit dans l'Exemple 3). Les composés obtenus présentaient des données analytiques identiques à celles de leurs énantiomères correspondants décrits dans l'Exemple 2, à l'exception des angles de rotation optique, présentés ci-après.

(-)-(S)-2,2-diméthyl-3-méthylèn-1-cyclohexaneacétaldéhyde

$[\alpha]_{D}^{20}$ (pur) = -21,6°

(-)-(1'S,E)-5-(2',2'-diméthyl-3'-méthylène-1'-cyclohexyl)-3-méthyl-3-pentén-2-one

$[\alpha]_{D}^{20}$ (pur) = -38,0°

(-)-(1'S,E)-5-(2',2'-diméthyl-3'-méthylène-1'-cyclohexyl)-3,3-diméthyl-4-pentén-2-one

$[\alpha]_{D}^{20}$ (pur) = -52,6°

(-)-(1'S,E)-5-(2',2'-diméthyl-3'-méthylène-1'-cyclohexyl)-3,3-diméthyl-4-pentén-2-ol

$[\alpha]_D^{20}$ (pur) = -64,6°
Odeur : santalée.

Exemple 5

Parfumage d'un savon

100 G de savon en copeaux, obtenu à partir d'une base de savon au sodium non-parfumée preparée avec de l'huile de coco et de suif, ont été mélangés avec 0,5 g de (-)-(1'R,E)-3,3-diméthyl-5-(2',2',3'-triméthyl-3'-cyclohexén-1'-yl)-4-pentén-2-ol d'une part, et de (+)-(1'R,E)-5-(2',2'-diméthyl-3'-méthylène-1'-cyclohexyl)-3,3-diméthyl-4-pentén-2-ol d'autre part, jusqu'à l'obtention de pâtes homogènes. Les compositions de savon ainsi obtenues possédaient, dans le premier cas une odeur à la fois santalée et ambrée et, dans le deuxième cas une odeur boisée, type bois de santal naturel.

Exemple 6

Composition parfumante

On a préparé une composition parfumante de base par mélange des ingrédients suivants :

| Ingrédients | Parties en poids |
|---|---|
| Décanal à 10%* | 10 |
| Aldéhyde undecylénique à 10%* | 40 |
| Dodécanal à 10%* | 10 |
| Aldéhyde méthylnonylacétique à 10%* | 10 |
| Essence d'armoise | 10 |
| Essence de castoréum à 10%* | 20 |
| Essence de civette à 10%* | 10 |
| Essence de galbanum résinoïde | 10 |
| Jasmin abs. à 50%* | 100 |
| Essence d'oliban | 10 |
| Essence de patchouli | 30 |
| Acétate de styrallyle | 15 |
| $\alpha$-Isométhylionone | 95 |
| Essence de coriandre | 5 |
| Levocitronellol | 50 |
| Exaltolide® à 10%* [1)] | 50 |
| Jasmin synth. | 100 |
| Bergamote synth. | 100 |
| Essence de citron de Sicile | 40 |
| Mousse de chêne abs. à 50%* | 30 |
| Neroli synth. | 20 |
| Galaxolide® 50 [2)] | 50 |

(continued)

| Ingrédients | Parties en poids |
|---|---|
| Coumarine | 50 |
| Tonalide® 3) | 40 |
| Dihydromyrcénol 4) | 75 |
| Total | 980 |

* dans le dipropylèneglycol (DIPG)
1) cyclopentadècanolide ; origine : Firmenich SA, Genève, Suisse
2) 1,3,4,6,7,8-hexahydro-4,6,6,7,8,8-hexamèthyl-cyclopenta[g]isochromène ; origine : International Flavors & Fragrances Inc., USA
3) 7-acètyl-1,1,3,4,4,6-hexamèthyltètraline ; origine : PFW, Hollande
4) 2,6-dimèthyl-7-octèn-2-ol ; origine : International Flavors & Fragrances Inc., USA.

Lorsqu'on a ajouté à 98 g de cette composition de base de type bois-animal 2 g de (+)-(1'R,E)-5-(2',2'-diméthyl-3'-méthylène-1'-cyclohexyl)-3,3-diméthyl-4-pentén-2-ol on a obtenu une nouvelle composition qui développait une odeur dont le caractère bois de santal était bien perceptible. D'autre part, lorsque l'on a remplacé le composé selon l'invention susmentionné par le (-)-(1'R,E)-3,3-diméthyl-5-(2',2',3'-triméthyl-3'-cyclohexén-1'-yl)-4-pentén-2-ol on a obtenu une composition nouvelle dont l'odeur avait un caractère moins santalé mais nettement plus ambré que celui de la composition nouvelle précédente.

Dans les deux cas, il a été trouvé que les composés selon l'invention remplaçaient avantageusement l'essence de santal naturelle (provenance Mysore) dont il aurait fallu employer une quantité double pour obtenir un effet similaire. L'utilisation de ces composés est ainsi d'autant plus avantageuse que l'essence de santal naturelle est très onéreuse et rare.

Exemple 7

Composition parfumante

On a préparé une composition parfumante de base en mélangeant les ingrédients suivants:

| Ingrédients | Parties en poids |
|---|---|
| Acétate de p-tert-butylcyclohexyle | 150 |
| Cedroxyde® 1) | 100 |
| Essence d'orange de Floride | 100 |
| Acétate de dihydroterpényle | 70 |
| α-Méthylionone | 60 |
| Essence de géranium Bourbon | 50 |
| Essence de lavande | 40 |
| Vertofix coeur 2) | 40 |
| Mayol® 3) | 40 |
| Florol® 4) | 40 |
| Musc cétone | 30 |
| Isoeugénol | 30 |
| Ambrox® DL à 10%* 5) | 30 |
| Acétate de myrcényle 6) | 30 |
| Rosinol | 20 |
| Salicylate d'amyle | 20 |
| Acétate TCD 7) | 20 |
| Bergamote synth. | 10 |
| Acétate de cyclohexyléthanol | 20 |
| Mousse de cristal à 10%* | 20 |
| Neroli synth. | 10 |
| β-Damascénone à 1%* | 10 |
| Dihydromyrcénol 8) | 10 |
| Total | 950 |

* dans le DIPG
1) triméthyl cyclododécatriéne époxyde ; origine : Firmenich SA, Genéve, Suisse
2) origine : International Flavors & Fragrances, Inc., USA
3) hydroxyméthyl isopropyl cyclohexane ; origine : Firmenich SA, Genéve, Suisse
4) tétrahydro-2-isobutyl-4-méthyl-4(2H)-pyranol ; origine : Firmenich SA, Genéve, Suisse
5) tétraméthyl perhydronaphtofurane ; origine : Firmenich SA, Genéve, Suisse
6) acétate de 2-méthyl-6-méthyléne-7-octén-2-yl ; origine : International Flavors & Fragrances, Inc., USA
7) acétate de (tricyclo[5.2.1.$0^{2,6}$]déc-4-yl)méthyle ; origine : Firmenich SA, Genéve, Suisse
8) voir Exemple 6

Avec cette composition de base de type fleuri-boisé, on a préparé quatre compositions, selon le tableau suivant :

TABLEAU

| Ingrédients | Composition / Poids en g | | | |
|---|---|---|---|---|
| | A | B | C | D |
| Base | 95 | 95 | 95 | 95 |
| (-)-(1'R,E)-3,3-diméthyl-5-(2',2',3'-triméthyl-3'-cyclohexén-1'-yl)-4-pentén-2-ol | 5 | -- | -- | -- |
| (+)-(1'R,E)-5-(2',2'-diméthyl-3'-méthylène-1'-cyclohexyl)-3,3-diméthyl-4-pentén-2-ol | -- | 5 | -- | -- |
| POLYSANTOL® | -- | -- | 5 | -- |
| BACDANOL® 1) | -- | -- | -- | 5 |

1) 2-éthyl-4-(2,2,3-triméthyl-3-cyclopentén-1-yl)-2-butén-1-ol, de IFF Inc.

Ces quatre compositions A à D on été évaluées à l'aveugle par un panel de 8 experts parfumeurs. Ces derniers ont préféré la composition A à l'unanimité pour la supériorité de sa note olfactive, aussi bien du point de vue du caractère de celle-ci que de sa puissance. D'autre part, la composition C a été généralement préférée à la composition B, laquelle a cependant été jugée olfactivement plus intéressante et agréable que la composition D.

**Revendications**

1. Composé de formule

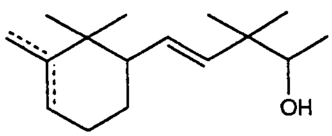

(I)

ayant une double liaison dans l'une des positions indiquées par les lignes pointillées.

2. Composé selon la revendication 1, sous forme d'un isomère optiquement actif de formule

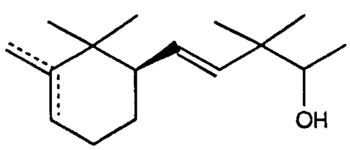

(Ia)

dans laquelle les lignes pointillées ont le sens indiqué à la formule (I), ou de son énantiomère correspondant.

3. (-)-(1'R,E)-3,3-Diméthyl-5-(2',2',3'-triméthyl-3'-cyclohexén-1'-yl)-4-pentén-2-ol.

4. Utilisation d'un composé selon l'une des revendications 1 à 3 à titre d'ingrédient parfumant.

5. Composition parfumante contenant à titre d'ingrédient parfumant un composé selon l'une des revendications 1 à 3.

6. Produit parfumé contenant à titre d'ingrédient parfumant un composé selon l'une des revendications 1 à 3.

7. Produit parfumé selon la revendication 6, sous forme d'un parfum ou d'une eau de toilette, d'un savon, d'un gel de douche ou bain, d'un shampoing ou d'un après-shampoing, d'une préparation cosmétique, d'un désodorisant corporel ou d'air ambiant, d'un détergent, d'un adoucissant textile ou d'un produit d'entretien.

8. Procédé pour la préparation d'un composé de formule

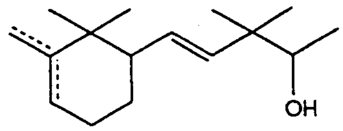

(I)

ayant une double liaison dans l'une des positions indiquées par les lignes pointillées, caractérisé en ce qu'on fait réagir un composé de formule

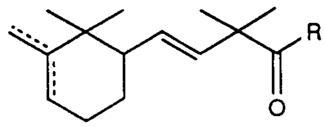

(II)

dans laquelle les lignes pointillées ont le sens indiqué à la formule (I) et R représente un atome d'hydrogène ou un radical méthyle, soit avec un agent réducteur, dans un solvant organique inerte, lorsque R est un radical méthyle, soit avec un halogéno-magnésien de méthyle lorsque R est un atome d'hydrogène.

**9.** Procédé selon la revendication 8, caractérisé en ce qu'on utilise en tant que produit de départ un composé optiquement actif de formule

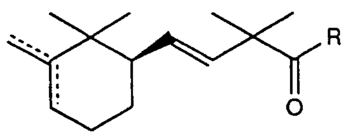

(IIa)

dans laquelle les lignes pointillées et le symbole R ont le sens indiqué à la formule (II), ou son énantiomère correspondant pour obtenir un composé de formule

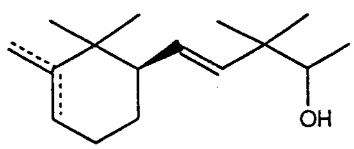

(Ia)

dans laquelle les lignes pointillées ont le sens indiqué à la formule (I), ou respectivement son énantiomère correspondant.

**10.** Composé de formule

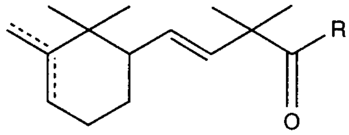

(II)

ayant une double liaison dans l'une des positions indiquées par les lignes pointillées et dans laquelle le symbole R représente un atome d'hydrogène ou un radical méthyle.

**11.** Composé selon la revendication 10, sous forme d'un isomère optiquement actif de formule

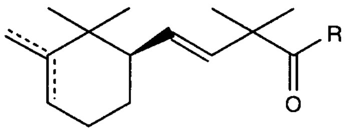

(IIa)

dans laquelle les lignes pointillées et le symbole R ont le sens indiqué à la formule (II), ou de son énantiomère correspondant.

**12.** Composé de formule

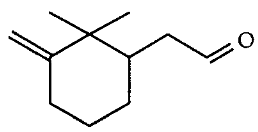

(III)

**13.** Composé optiquement actif de formule

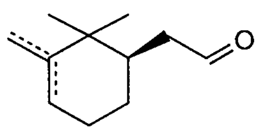

(IIIa)

possédant une double liaison dans l'une des positions indiquées par les lignes pointillées, ou de son énantiomère correspondant.

**Claims**

**1.** Compound of formula

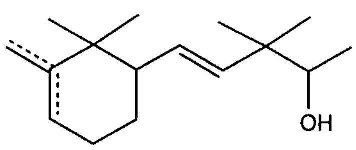

(I)

having a double bond in one of the positions indicated by the dotted lines.

**2.** Compound according to claim 1, in the form of an optically active isomer of formula

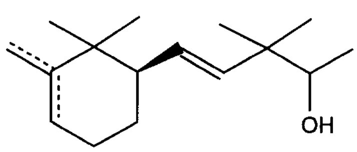

(Ia)

wherein the dotted lines have the meaning indicated in formula (I), or of its corresponding enantiomer.

**3.** (-)-(1'R,E)-3,3-Dimethyl-5-(2',2',3'-trimethyl-3'-cyclohexen-1'-yl)4-penten-2-ol.

4. Use of a compound according to any one of claims 1 to 3 as a perfuming ingredient.

5. Perfuming composition containing as perfuming ingredient a compound according to any one of claims 1 to 3.

6. Perfumed product containing as perfuming ingredient a compound according to any one of claims 1 to 3.

7. Perfumed product according to claim 6, in the form of a perfume or a cologne, a soap, a shower or bath gel, a shampoo or an after-shampoo product, a cosmetic preparation, a body or air deodorant, a detergent, a fabric softener or a household product.

8. Process for the preparation of a compound of formula

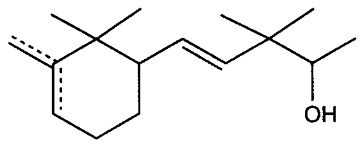

(I)

having a double bond in one of the positions indicated by the dotted lines, characterized by the reaction of a compound of formula

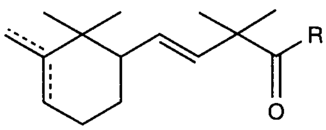

(II)

wherein the dotted lines have the meaning indicated in formula (I) and R represents a hydrogen atom or a methyl radical, either with a reducing agent, in an inert organic solvent, when R is a methyl radical, or with a methyl halomagnesium reagent when R is a hydrogen atom.

9. Process according to claim 8, characterized in that, as starting product, there is used an optically active compound of formula

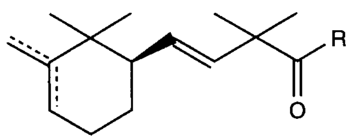

(IIa)

wherein the dotted lines and symbol R have the meaning indicated in formula (II), or its corresponding enantiomer, to obtain a compound of formula

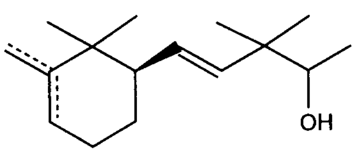

(Ia)

wherein the dotted lines have the meaning indicated in formula (I), or respectively its corresponding enantiomer.

10. Compound of formula

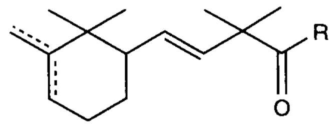

(II)

having a double bond in one of the positions indicated by the dotted lines and wherein symbol R represents a hydrogen atom or a methyl radical.

11. Compound according to claim 10, in the form of an optically active isomer of formula

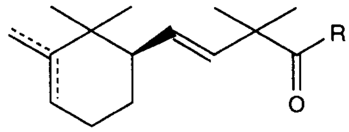

(IIa)

wherein the dotted lines and symbol R have the meaning indicated in formula (II), or of its corresponding enantiomer.

12. Compound of formula

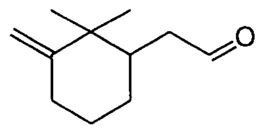

(III)

13. Optically active compound of formula

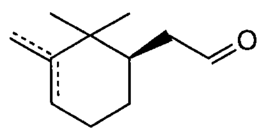

(IIIa)

having a double bond in one of the positions indicated by the dotted lines, or its corresponding enantiomer.

**Patentansprüche**

1. Verbindung der Formel

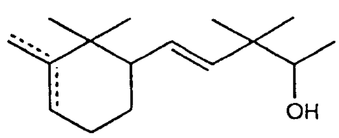

(I)

, welche eine Doppelbindung in einer durch die punktierten Linien angegebenen Stellungen besitzt.

2. Verbindung gemäss Patentanspruch 1, in Form eines optisch aktiven Isomeren der Formel

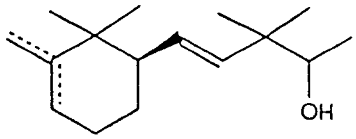

(Ia)

, worin die punktierten Linien die in der Formel (I) angegebene Bedeutung besitzen, oder ihres entsprechenden Enantiomeren.

3. (-)-(1'R,E)-3,3-Dimethyl-5-(2',2',3'-trimethyl-3'-cyclohexen-1'-yl)-4-penten-2-ol.

4. Verwendung einer Verbindung gemäss einem der Patentansprüche 1 bis 3 als Parfümbestandteil.

5. Riechstoffzusammensetzung, enthaltend als Riechstoffbestandteil eine Verbindung gemäss einem der Patentansprüche 1 bis 3.

6. Parfümiertes Produkt, enthaltend als Riechstoffbestandteil eine Verbindung gemäss einem der Patentansprüche 1 bis 3.

7. Parfümiertes Produkt gemäss Patentanspruch 6, in Form eines Parfüms oder Toilettwassers, einer Seife, eines Bade- oder Douchegels, eines Shampoos oder einer Nachshampoospülung, einer kosmetischen Zubereitung, eines Körperdesodorans oder eines Raumluftverbesserers, eines Reinigungsmittels, eines Textilauffrischungsmittels oder eines Pflegemittels.

8. Verfahren zur Herstellung einer Verbindung der Formel

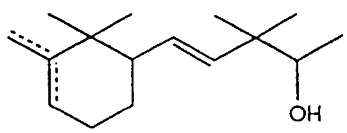

(I)

, welche eine Doppelbindung in einer der durch die punktierten Linien angegebenen Stellungen besitzt, dadurch gekennzeichnet, dass man eine Verbindung der Formel

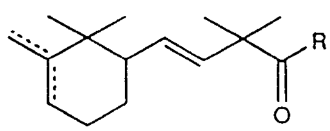

(II)

, worin die punktierten Linien die in der Formel (I) angegebene Bedeutung besitzen und R ein Wasserstoffatom oder einen Methylrest bedeutet, entweder, falls R einen Methylrest bedeutet, mit einem Reduktionsmittel in einem inerten organischen Lösungsmittel oder, falls R ein Wasserstoffatom bedeutet, mit einem Methylmagnesiumhalogenid umsetzt.

9. Verfahren gemäss Patentanspruch 8, dadurch gekennzeichnet, dass man als Ausgangsverbindung eine optisch aktive Verbindung der Formel

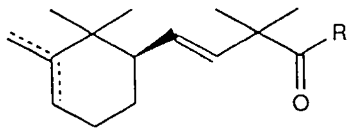

(IIa)

, worin die punktierten Linien und das Symbol R die in der Formel (II) angegebene Bedeutung besitzen, oder ihr entsprechendes Enantiomeres verwendet, um eine Verbindung der Formel

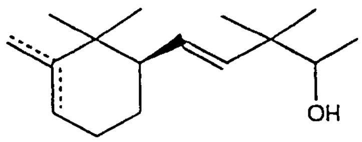

(Ia)

zu erhalten, worin die punktierten Linien die in der Formel (I) angegebene Bedeutung besitzen, oder ihr entsprechendes Enantiomeres.

**10.** Verbindung der Formel

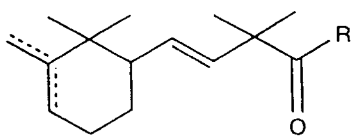

(II)

, welche eine Doppelbindung in einer der durch die punktierten Linien angegebenen Stellungen besitzt und worin das Symbol R für ein Wasserstoffatom oder einen Methylrest steht.

**11.** Verbindung gemäss Patentanspruch 10, in Form eines optisch aktiven Isomeren der Formel

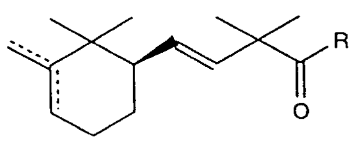

(IIa)

, worin die punktierten Linien und das Symbol R die in der Formel (II) angegebene Bedeutung besitzen, oder ihres entsprechenden Enantiomeren.

**12.** Verbindung der Formel

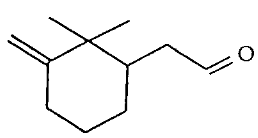

(III)

**13.** Optisch aktive Verbindung der Formel

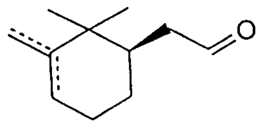

(IIIa)

, welche eine Doppelbindung in einer der durch die punktierten Linien angegebenen Stellungen besitzt, oder ihr entsprechendes Enantiomeres.